# EUROPÄISCHE PATENTSCHRIFT

(11) **EP 2 527 000 B1**
(45) Veröffentlichungstag und Bekanntmachung des Hinweises auf die Patenterteilung: **25.11.2015**
(21) Anmeldenummer: 12159231.5
(22) Anmeldetag: 13.03.2012
(51) Int. Cl.: H01G 9/008, H01G 11/74, H01G 11/78, H01G 11/80, H01M 2/06, H01M 2/08, H01G 9/10, A61N 1/375

(54) **Leitungs-Durchführung und elektrische Funktionseinheit**
Lead feedthrough and electrical functional unit
Passage de conduite et unité de fonctionnement électrique

(30) Priorität: 15.04.2011 US 201161475685 P
(43) Veröffentlichungstag der Anmeldung: 28.11.2012
(60) Teilanmeldung: 14189825.4
(73) Patentinhaber: LITRONIK Entwicklungs GmbH, 25813 Husum (DE)
(72) Erfinder: Pretzlaff, Bernd, 25866 Milstedt (DE); Kühl, Hans-Jürgen, 25774 Lunden (DE)
(74) Vertreter: Galander, Marcus

(56) Entgegenhaltungen:
- US-A- 5 930 109
- US-A1- 2002 142 216
- US-A1- 2004 260 354
- US-A1- 2010 134 955
- US-B1- 6 850 405

## Beschreibung

Die Erfindung betrifft eine Leitungs-Durchführung einer elektrischen Funktionseinheit, insbesondere eines Kondensators oder medizinelektronischen Implantats, welche einen hohlen leitenden Flansch und ein in dessen Hohlraum angeordnetes und gegenüber dem Flansch elektrisch isoliertes und abgedichtetes Kontaktstück aufweist, wobei der Flansch mit einem Leiter eines ersten Typs und das Kontaktstück mit einem Leiter eines zweiten Typs in der Funktionseinheit verbindbar ist.

Bei elektronischen Geräten und elektrischen Bauteilen, die besonderen Anforderungen hinsichtlich der dauerhaften Dichtigkeit genügen müssen, sind die sogenannten Durchführungen um einen Anschlussleiter besonders kritische Bereiche. Ihrer Gestaltung wird daher bei solchen Geräten, etwa bei implantierbaren medizin-elektronischem Geräten (Schrittmachern, implantierbaren Kardiovertern, Cochlearimplantaten, implantierbaren Neurostimulatoren etc.) seit Jahren besondere Aufmerksamkeit geschenkt. Technisch etabliert ist dabei die Bildung der Durchführung aus geeigneter Keramik, speziell umgeben von einem metallischen Flansch des Gehäuses.

Speziell für elektrochemische Bauteile, wie etwa Batterien oder Elektrolyt-Kondensatoren, ist aus der WO 2005/001997 A2 eine Durchführung bekannt, bei der ein Führungselement aus einem Polymeren zur Aufnahme des Anschlussleiters vorgesehen ist.
Aus der WO 2009/015438 A1 sind ein Verfahren und eine Vorrichtung zur Bildung einer Leitungs-Durchführung aus einem hermetisch abgedichteten Behälter bekannt, welche sich dadurch auszeichnen, dass Mittel bzw. Schritte zum Einspritzen eines elektrisch isolierenden Materials in einen Hohlraum der Durchführung bereitgestellt werden.

Aus der US 2010/0134955 A1 ist eine Leitungs-Durchführung eines Mehranoden-Kondensators bekannt, die den eingangs beschriebenen Grundaufbau hat. Hermetische Dichtigkeit wird hier durch eine Glas-Metall-Versiegelung bewirkt, und es ist ein Anschweißen eines folienartigen Leiterabschnitts des Kondensators an die Außenoberfläche des Flansches der Durchführung per Laserschweißen vorgesehen.
US 2002/0142216 A1 beschreibt eine Leitungs-Durchführung bei der das Einpressen eines zweiten Leiters zwischen Gehäuserand und Deckel zur Fixierung des Leiters führt.

Der Erfindung liegt die Aufgabe zugrunde, eine verbesserte Leitungs-Durchführung der genannten Art bereitzustellen, welche insbesondere für schwierig zu handhabende Leiter-Anschlüsse relativ leicht und mit hoher Zuverlässigkeit herstellbar ist.

Diese Aufgabe wird durch eine Leitungs-Durchführung mit den Merkmalen des Anspruchs 1 gelöst. Zweckmäßige Fortbildungen des Erfindungsgedankens sind der Gegenstand der abhängigen Ansprüche. Im Zusammenhang mit der Erfindung werden auch eine elektrische Funktionseinheit mit einer erfindungsgemäßen Durchführung bereitgestellt.

Darüberhinaus wird ein Verfahren zur Herstellung einer Leitungs-Durchführung beschrieben. Das Verfahren ist jedoch nicht Teil der Erfindung.

Ein Aspekt der Erfindung ist es, in einem Umfangsabschnitt des Flansches einen Spalt zum Einschieben und Fixieren von dort anzuschließenden Leitern der elektrischen Funktionseinheit vorzusehen. Gemäß einem weiteren Aspekt erstreckt dieser Spalt sich tangential und wird auf einer Seite von einem Flansch-Grundkörper begrenzt. Gemäß einem weiteren Aspekt der Erfindung wird der Spalt auf der anderen Seite von einer sich beabstandet zum Flansch-Grundkörper erstreckenden und an diesen angeformten Zunge.

Gemäß einer ersten Ausführung der Erfindung ist der Spalt zum tangentialen Einschieben und zur Fixierung eines oder insbesondere mehrerer als Blech oder Folie ausgeführter Leiter des ersten Typs flach quaderförmig ausgebildet. Speziell ist hierbei die Zunge oder das Andruckstück flach prismatisch, insbesondere als flacher Quader oder flaches Trapezoid ausgebildet. Diese Ausführung ist speziell dann sinnvoll, wenn der Flansch-Grundkörper im wesentlichen prismatisch geformt ist oder zumindest eine plane Umfangsfläche hat, welche zur Kontaktierung des besagten Leiters oder der Leiter des ersten Typs vorgesehen ist.

In einer hierzu alternativen Ausführung ist der Spalt zum tangentialen Einschieben und zur Fixierung eines oder insbesondere mehrerer als Blech oder Folie ausgeführter Leiter des ersten Typs flach ringsegmentförmig ausgebildet. Speziell weist hierbei die Zunge einen Hohlzylinderabschnitt auf. Diese konstruktive Ausgestaltung ist sinnvoll bei einem Flansch-Grundkörper mit im Wesentlichen zylindrischer Außenform.

In einer weiteren konstruktiven Ausgestaltung ist vorgesehen, dass der Flansch eine über seinen übrigen Umfang sowie die maximale radiale Erstreckung der Zunge überstehende Stirnfläche aufweist. Dies ist bei bestimmten Gehäusekonstruktionen der elektrischen Funktionseinheit vorteilhaft zur Erreichung einer exakten Positionierung der Durchführung und kann eine mechanische Schutzfunktion erfüllen.

Gemäß dem weiter oben erwähnten Beispiel ist der Hohlraum des Flansches mit einem Pfropf aus spritzfähigem Kunststoff gefüllt, der das Kontaktstück dicht umschließt und ebenso dicht an der Innenwandung des Flansches anliegt. Hierdurch wird auf technologisch höchst einfache und produktive Weise ein hoher Grad an Dichtigkeit des Gehäuses im Bereich der Durchführung gesichert. Zur Erreichung größerer Kriechstrecken und/oder größerer Haftungsflächen ist in Ausgestaltungen dieses Aspektes vorgesehen, dass der Kunststoff-Pfropf sich über die axiale Erstreckung des Flansches hinaus erstreckt und insbesondere seine radiale Erstreckung im überstehenden Abschnitt größer als diejenige des Flansches ist.

Bei der mit einer erfindungsgemäßen Durchführung versehenen elektrischen Funktionseinheit ist bzw. sind, gemäß einer weiteren Ausführung der Erfindung, der oder die Leiter des ersten Typs im oder am Spalt mit dem Flansch verschweißt.

Speziell bei einer Ausführung der Funktionseinheit als Kondensator ist eine Vielzahl von als Katodenfahnen ausgebildeten Leitern des ersten Typs vorgesehen, die übereinander in den Spalt eingelegt und dort miteinander und/oder mit dem Flansch verschweißt sind. Obgleich ein derartiger Kondensator eine bevorzugte Anwendungsmöglichkeit der Erfindung bietet, ist deren Anwendung nicht auf Kondensatoren Beschränkt, sondern auch bei anderen elektrischen oder elektronischen Geräten oder Bauteilen möglich, darunter insbesondere auch bei elektromedizinischen Implantaten wie Schrittmachern und implantierbaren Defibrillatoren oder Kardiovertern. Es versteht sich, dass der Fachmann die konkrete konstruktive Ausgestaltung der vorgeschlagenen Durchführung in Abstimmung auf die spezifische Leiter-Konfiguration des jeweiligen Gerätes bestimmen wird.

Im Verfahren zur Herstellung der Leitungs-Durchführung ist vorgesehen, dass der oder die Leiter des ersten Typs in den Spalt eingesetzt und durch Andrücken der Zunge oder des Kontaktstücks an den Flansch-Grundkörper kalt verschweißt werden. Eine alternative Ausgestaltung sieht vor, dass der oder die Leiter des ersten Leiter-Typs in den Spalt eingesetzt und ihre freien Enden durch Laserschweißen verbunden werden.

Eine weitere Beschreibung des Verfahrens zur Herstellung der Leitungs-Druchführung sieht vor, dass der Kunststoff-Pfropf im Hohlraum des Flansches durch Spritzgießen erzeugt wird, wobei der Flansch und das Kontaktstück in vorgesehener Lagebeziehung zueinander in ein Spritzgießwerkzeug eingelegt werden. Grundsätzlich sind hierzu auch Alternativen denkbar, wie etwa das Einfügen eines geeignet kompressiblen und dennoch gasdichten vorgefertigten Kunststoffpfropfens in den Flansch.

Vorteile und Zweckmäßigkeiten der Erfindung ergeben sich im Übrigen aus den nachfolgenden Beschreibung von Ausführungsbeispielen anhand der Figuren. Von diesen zeigen:
- Fig. 1: eine schematische perspektivische Darstellung einer elektrischen Funktionseinheit mit einer Durchführung,
- Fig. 2: eine perspektivische Darstellung einer ersten Ausführungsform der erfindungsgemäßen Durchführung,
- Fig. 3: eine perspektivische Darstellung einer zweiten Ausführungsform der erfindungsgemäßen Durchführung,
- Fig. 4A bis 4F: schematische Querschnittsdarstellungen bzw. Draufsichten auf Flansch-Kontaktstück-Anordnungen in Ausführungsbeispielen der erfindungsgemäßen Durchführung, und
- Fig. 5: eine perspektivische Darstellung einer weiteren Ausführungsform der erfindungsgemäßen Durchführung.

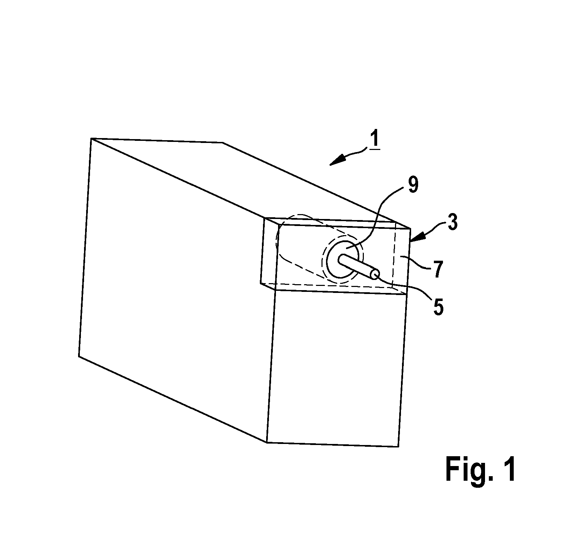

In Fig. 1 ist schematisch eine elektrische Funktionseinheit 1 mit einer in einem Eckenbereich eingesetzten Durchführung 3 gezeigt, welche ein zentral herausragendes Kontaktstück 5, einen das Kontaktstück umgebenden Flansch 7 mit rechteckiger Stirnfläche sowie einen das Kontaktstück gegenüber dem Flansch elektrisch isolierenden und abdichtenden Kunststoffpfropfen 9 aufweist. Es kann sich hierbei bspw. um einen Kondensator oder ein implantierbares medizinelektronisches Gerät handeln.

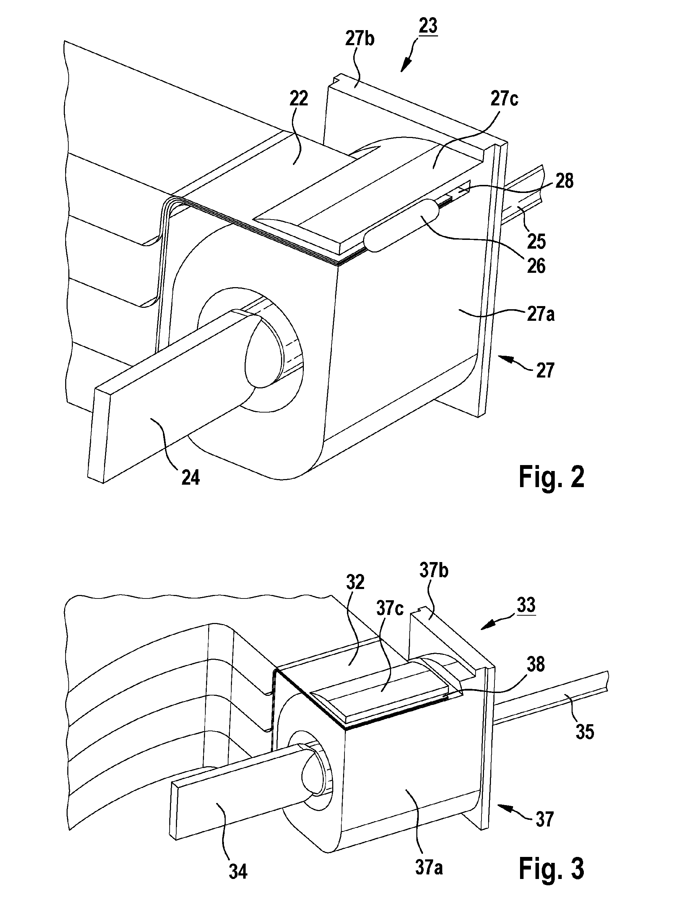

Fig. 2 zeigt eine Durchführung 23 eines (nicht insgesamt dargestellten) Kondensators, von dem eine Mehrzahl von Katodenfahnen 22 gezeigt ist. Die Bezugsziffern zur Bezeichnung von Teilen der Durchführung gemäß Fig. 2 sind an die in Fig. 1 verwendeten Bezugsziffern angelehnt. Die Katodenfahnen 22 stellen einen Leiter ersten Typs dar, der mit dem Flansch 27 der Durchführung verbunden ist, während mit dem Kontaktstück 25 ein quaderförmiger Leiter (Leiter zweiten Typs) 24 verbunden ist. Der Flansch 27 umfasst einen Grundkörper 27a, eine über dessen radiale Erstreckung teilweise überstehende Stirnfläche 27b und eine sich von der Stirnfläche aus parallel zum Grundkörper erstreckende flache Zunge 27c.
Die Zunge 27c ist derart an die Stirnfläche 27b angeformt, dass zwischen ihr und dem Flansch-Grundkörper 27a ein im Wesentlichen quaderförmiger Spalt 28 bestimmt wird, in den die Enden einer Vielzahl von Katodenfahnen 22 tangential eingeschoben werden können, die auf die in der Figur gezeigte Weise um einen Teil des Flansch-Grundkörpers 27a herumgelegt sind. Mit Ziffer 26 ist schematisch ein durch Laserschweißen gebildeter Verschweißungsbereich der Katodenfahnen 22 mit dem Flansch 27 dargestellt. In dem Verschweißungsbereich 26 sind die vorher in den Spalt 28 eingesetzten und dort vorläufig fixierten Katodenfahnen (Leiter ersten Typs) fest mit dem Flansch als äußerem Teil der Durchführung 23 verbunden.

Fig. 3 zeigt als modifizierte Ausführung eine Durchführung 33. Deren Aufbau gleicht weitgehend demjenigen der Durchführung 23 nach Fig. 2, sodass zur Bezeichnung der einzelnen Teile an Fig. 2 angelehnte Bezugsziffern verwendet wurden und die übereinstimmenden oder einander entsprechenden Teile hier nicht nochmals erläutert werden. Des wesentliche Unterschied besteht darin, dass anstelle einer an den Flansch-Grundkörper angeformten Zunge hier ein separates Andruckstück 37c vorgesehen ist, welches zwischen sich und dem Flansch-Grundkörper 37a den Spalt 38 zum Einsetzen der Katodenfahnen 32 bestimmt, und dass durch Ausübung entsprechend hohen Drucks auf das Andruckstück 37c ein Kaltverschweißen mit den Katodenfahnen 32 und dem Flansch-Grundkörper 37 (anstelle der in Fig. 2 dargestellten Laserverschweißung) erfolgt.
In einer weiteren Ausführung wird Widerstandsschweißen zum Herstellen der Verbindung verwendet.

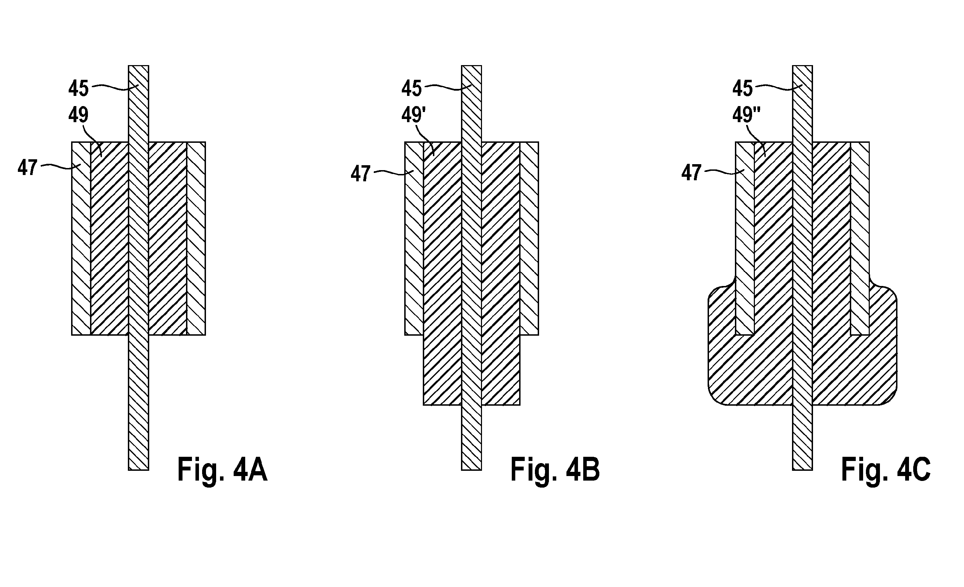

Die Figuren 4A bis 4F zeigen verschiedene Varianten der geometrischen Ausbildung des Flansches sowie eines den Flansch ausfüllenden und das darin angeordnete Kontaktstück umgebenden Kunststoff-Pfropfes.

Während bei den Ausführungen nach Fig. 4A bis 4C die Kontaktstücken 45 und Flansche 47 identisch sind, sind drei verschiedene Kunststoff-Pfropfen 49, 49' bzw. 49" vorgesehen. Der Kunststoff-Pfropf 49' gemäß Fig. 4B erstreckt sich über die Längserstreckung des Flansches 47 hinaus, behält aber im überstehenden Teil der Innendurchmesser des Flansches als eigenen Außendurchmesser bei. Bei der Ausführung nach Fig. 4C ist der Kunststoff-Pfropf 49" gegenüber dem Flansch 47 ebenfalls verlängert, weist über annähernd die Hälfte seiner Längserstreckung aber auch einen vergrößerten Durchmesser auf, derart, dass ein Ende des Flansches in den Bereich vergrößerten Durchmessers des Kunststoff-Pfropfes hineinragt und in diesen eingebettet ist.

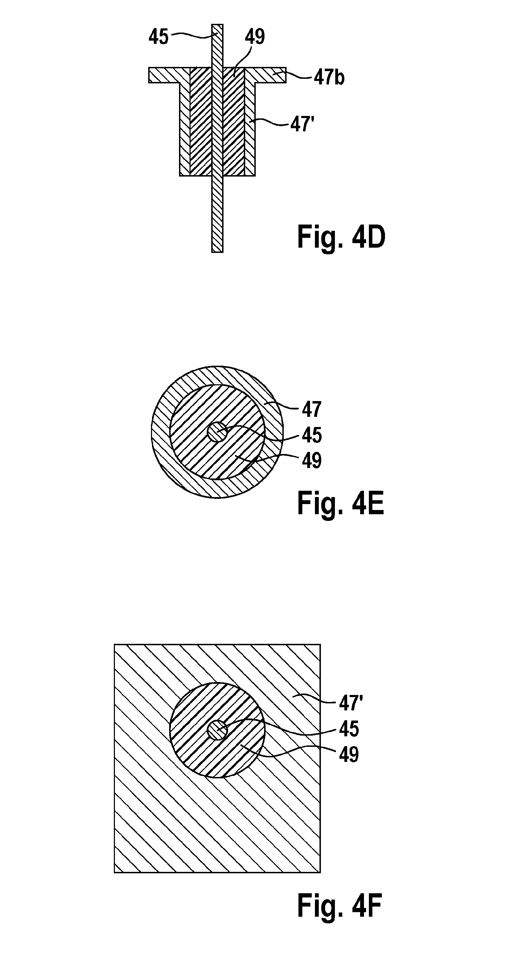

Fig. 4D zeigt einen Flansch 47' mit einem angeformten Bund bzw. einer Stirnfläche 47b. Während die Stirnseite des in Fig. 4A gezeigten Aufbaus in Fig. 4E gezeigt ist, kann die Draufsicht auf einen mit Stirnfläche versehenen Flansch (bspw. den in Fig. 4E gezeigten) aussehen wie in Fig. 4F.

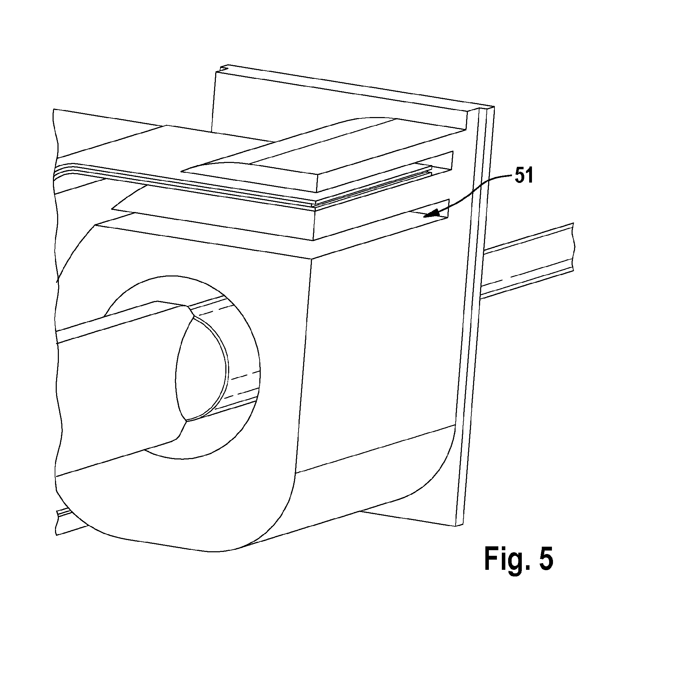

Fig. 5 zeigt eine weitere Ausführungsform, bei der ein zweiter Schlitz 51 vorhanden ist. Dieser Schlitz 51 sorgt dafür, dass die Abdichtung durch das Schweißen der Anschlussfahnen weniger stark erhitzt wird.

Die Ausführung der Erfindung ist nicht auf die oben beschriebenen Beispiele und hervorgehobenen Aspekte beschränkt, sondern kann ebenso in einer Vielzahl von Abwandlungen erfolgen, die im Rahmen fachmännischen Handelns liegen. Insbesondere kann die Abdichtung durch eine Kombination von Glas bzw. Keramik und Kunststoff erfolgen.

## Patentansprüche

1. Leitungs-Durchführung einer elektrischen Funktionseinheit (1), insbesondere eines Kondensators oder medizinelektronischen Implantats, welche einen hohlen leitenden Flansch (7, 27) und ein in dessen Hohlraum angeordnetes und gegenüber dem Flansch elektrisch isoliertes und abgedichtetes Kontaktstück (5, 25) aufweist, wobei der Flansch mit einem Leiter eines ersten Typs (22) und das Kontaktstück (5, 25)mit einem Leiter eines zweiten Typs (24) in der Funktionseinheit (1) verbindbar ist, wobei in einem Umfangsabschnitt des Flansches (7, 27) ein sich tangential erstreckender, auf einer Seite von einem Flansch-Grundkörper (27a) und auf der anderen Seite von einer an den Grundkörper (27a) angeformten Zunge (27c) begrenzter Spalt (28) zum tangentialen Einschieben und Fixieren des Leiters des ersten Typs (22) vorgesehen ist.

2. Leitungs-Durchführung nach Anspruch 1, wobei der Spalt zum tangentialen Einschieben und zur Fixierung eines oder insbesondere mehrerer als Blech oder Folie ausgeführter Leiter des ersten Typs (22) flach quaderförmig ausgebildet ist.

3. Leitungs-Durchführung nach Anspruch 2, wobei die Zunge (27c) oder das Andruckstück (37c) flach prismatisch, insbesondere als flacher Quader oder flaches Trapezoid ausgebildet ist.

4. Leitungs-Durchführung nach einem der vorangehenden Ansprüche, wobei der Flansch (7, 27) eine über seinen übrigen Umfang sowie die maximale radiale Erstreckung der Zunge (27c) überstehende Stirnfläche (27b) aufweist.

5. Leitungs-Durchführung der vorangehenden Ansprüche, wobei der Hohlraum des Flansches mit einem Pfropf (9, 49) aus spritzfähigem Kunststoff gefüllt ist.

6. Leitungs-Durchführung nach Anspruch 5, wobei der Kunststoff-Pfropf (49")sich über die axiale Erstreckung des Flansches (7, 27) hinaus erstreckt und insbesondere seine radiale Erstreckung im überstehenden Abschnitt größer als diejenige des Flansches (7, 27) ist.

7. Elektrische Funktionseinheit mit einer Leitungs-Durchführung nach einem der vorangehenden Ansprüche, wobei der oder die Leiter des ersten Typs (22) im oder am Spalt mit dem Flansch verschweißt ist/sind.

8. Elektrische Funktionseinheit nach Anspruch 7, ausgebildet als Kondensator wobei eine Vielzahl von als Katodenfahnen ausgebildeten Leitern des ersten Typs (22) vorgesehen ist, die übereinander in den Spalt eingelegt und dort miteinander und/oder mit dem Flansch verschweißt sind.

## Claims

1. A lead feedthrough of an electrical functional unit (1), in particular of a capacitor or medical electronic implant, having a hollow conductive flange (7, 27) and a contact piece (5, 25) disposed in the cavity of said flange and electrically insulated and sealed with respect to the flange, wherein the flange can be connected to a lead of a first type (22) and the contact piece (5, 25) can be connected to a lead of a second type (24) in the functional unit (1), wherein a tangentially extending gap (28), which is delimited on one side by a flange main body (27a) and on the other side by a tab (27c) integrally formed on the main body (27a), is provided in a peripheral portion of the flange (7, 27) for the tangential insertion and fixation of the lead of the first type (22).

2. The lead feedthrough according to Claim 1, wherein the gap for the tangential insertion and fixation of one or in particular a plurality of leads of the first type (22) formed as a sheet or film has a flat cuboid shape.

3. The lead feedthrough according to Claim 2, wherein the tab (27c) or the pressure piece (37c) is configured in a flat prismatic shape, in particular as a flat cube or a flat trapezoid.

4. The lead feedthrough according to one of the preceding claims, wherein the flange (7, 27) has an end face (27b) extending past the remaining circumference of said flange and past the maximum radial extension of the tab (27c).

5. The lead feedthrough according to one of the preceding claims, wherein the cavity of the flange is filled with a plug (9, 49) of injectable plastic.

6. The lead feedthrough according to Claim 5, wherein the plastic plug (49") extends past the axial extension of the flange (7, 27) and in particular the radial extension of said plug is greater in the overhanging portion than that of the flange (7, 27).

7. An electrical functional unit comprising a lead feedthrough according to one of the preceding claims, wherein the lead or leads of the first type (22) is/are welded to the flange in or at the gap.

8. The electrical functional unit according to Claim 7, formed as a capacitor, wherein a plurality of leads of the first type (22) formed as cathode lugs are provided and are inserted one above the other into the gap and are welded there to one another and/or to the flange.

## Revendications

1. Passe-fils d'une unité fonctionnelle électrique (1), notamment d'un condensateur ou d'un implant électronique médical, lequel présente une bride conductrice creuse (7, 27) et une pièce de contact (5, 25) disposée dans son espace creux et isolée électriquement et de manière étanche par rapport à la bride, où la bride peut être reliée avec un conducteur d'un premier type (22) et la pièce de contact (5, 25) peut être reliée avec un conducteur d'un deuxième type (24) dans l'unité fonctionnelle (1),
dans lequel une fente (28), s'étendant de manière tangentielle dans une section circonférentielle de la bride (7, 27), limitée sur un côté par un corps de base de bride (27a) et sur l'autre côté par une languette (27c) moulée sur le corps de base (27a), est prévue pour une insertion tangentielle et une fixation du conducteur du premier type (22).

2. Passe-fils selon la revendication 1, dans lequel la fente pour l'insertion tangentielle et pour la fixation d'un ou, en particulier, de plusieurs conducteurs du premier type (22) réalisés sous la forme de tôles ou de films, est conçue sous une forme d'un cuboïde plan.

3. Passe-fils selon la revendication 2, dans lequel la languette (27c) ou la pièce d'appui (37c) est conçue prismatique plane, en particulier, sous la forme d'un cuboïde plan ou d'un trapézoïde plan.

4. Passe-fils selon l'une des revendications précédentes, dans lequel la bride (7, 27) présente une surface frontale (27b) dépassant par son pourtour résiduel ainsi que par l'extension radiale maximale de la languette (27c).

5. Passe-fils selon l'une des revendications précédentes, dans lequel l'espace creux de la bride est rempli avec un bouchon (9, 49) constitué d'une matière synthétique pouvant être pulvérisée.

6. Passe-fils selon la revendication 5, dans lequel le bouchon en matière synthétique (49") s'étend au-delà de l'extension axiale de la bride (7, 27) et son extension radiale dans la partie en saillie est en particulier plus importante que celle de la bride (7, 27).

7. Unité fonctionnelle électrique avec un passe-fils selon l'une des revendications précédentes, dans laquelle le ou les conducteurs du premier type (22) sont soudés avec la bride dans ou sur la fente.

8. Unité fonctionnelle électrique selon la revendication 7, conçue sous la forme d'un condensateur, dans laquelle est prévue une multiplicité de conducteurs du premier type (22) réalisés sous la forme de lames de cathode, qui sont insérées les unes sur les autres dans la fente et y sont soudées ensemble et/ou avec la bride.
